# EUROPÄISCHE PATENTSCHRIFT

(11) **EP 1 694 477 B1**
(45) Veröffentlichungstag und Bekanntmachung des Hinweises auf die Patenterteilung: **15.08.2007**
(21) Anmeldenummer: 03819107.8
(22) Anmeldetag: 15.12.2003
(51) Int. Cl.: B27B 5/32

(54) **KUPPLUNGSVORRICHTUNG**
COUPLING DEVICE
DISPOSITIF D'ACCOUPLEMENT

(43) Veröffentlichungstag der Anmeldung: 30.08.2006
(73) Patentinhaber: Synthes GmbH, 4436 Oberdorf (CH)
(72) Erfinder: TANNER, Peter, CH-4416 Bubendorf (CH)
(74) Vertreter: Lusuardi, Werther
(86) Internationale Anmeldenummer: PCT/CH2003/000816
(87) Internationale Veröffentlichungsnummer: WO 2005/056256

(56) Entgegenhaltungen:
- DD-A- 221 385
- FR-A- 2 255 814
- US-A- 5 839 196
- US-A1- 2002 198 556

## Beschreibung

Die Erfindung bezieht sich auf eine Kupplungsvorrichtung gemäss dem Oberbegriff des Patentanspruchs 1. Eine solche Vorrichtung ist aus DD 221385 bekannt.

Rotativ oszillierende chirurgische Sägeblätter, wie beispielsweise mandibula, crescentic oder sagittale Sägeblätter werden wegen ihrer oszillierenden Bewegung vorzugsweise rotativ formschlüssig mit der Antriebswelle verbunden.

Aus der US 5,702,415 MATTHAI ist eine gattungsgemässe Kupplungsvorrichtung bekannt, welche Verriegelungsmittel zur lösbar fixierbaren Verbindung eines chirurgischen Sägeblattes mit einer Antriebsvorrichtung aufweist. Die Verriegelungsmittel umfassen parallel zur Längsachse der Antriebswelle angeordnete Führungsstifte, welche derart in Öffnungen im Sägeblatt einführbar sind, dass das Sägeblatt quer zur Längsachse und parallel zur Anlagefläche an der Antriebswelle verschiebbar ist. Wenn das Sägeblatt zentriert ist, rasten zwei ebenfalls parallel zur Längsachse angeordnete Stifte in die Öffnungen ein, so dass das Sägeblatt rotativ formschlüssig mit der Antriebswelle verbunden ist. Ferner ist an der Antriebswelle koaxial endständig eine Schraube angeordnet, deren Schraubenkopf beim Anziehen der Schraube axial gegen das Sägeblatt gepresst wird, so dass das Sägeblatt auch in axialer Richtung fixiert ist. Nachteilig an dieser bekannten Kupplungsvorrichtung ist, dass nur Sägeblätter, welche Einspannsegmente eines ganz bestimmten Typs aufweisen, in der Kupplungsvorrichtung aufnehmbar sind.

Hier will die Erfindung Abhilfe schaffen. Der Erfindung liegt die Aufgabe zugrunde, eine Kupplungsvorrichtung zu schaffen, welche eine Verbindung von verschiedenen Sägeblättern, welche unterschiedliche Einspannsegmente aufweisen, mit der Antriebswelle gestattet.

Die Erfindung löst die gestellte Aufgabe mit einer Kupplungsvorrichtung, welche die Merkmale des Anspruchs 1 aufweist.

Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass dank der erfindungsgemässen Kupplungsvorrichtung:
- Werkzeuge mit verschiedenen Einspannsegmenten einsetzbar sind; und
- Verschiedene Werkzeuge, beispielsweise schaftlose, ebene Sägeblätter mit peripher angeordneten Sägezähnen oder gebogene Sägeblätter mit einem Einspannschaft und stirnseitig bogenförmig angeordneten Sägezähnen in eine einzige Kupplungsvorrichtung einsetzbar sind.

In einer bevorzugten Ausführungsform ist die erste Einspannvorrichtung derart ausgebildet, dass die Einspannmittel eines Werkzeuges koaxial zur Längsachse einführbar sind. Damit ist der Vorteil erreichbar, dass handelsübliche chirurgische Werkzeuge, welche einen Einspannzapfen umfassen, in der Einspannvorrichtung einspannbar sind.

In einer anderen Ausführungsform ist die zweite Einspannvorrichtung derart ausgebildet, dass ein Werkzeug quer zur Längsachse einführbar ist. Auch hier ist der Vorteil erreichbar, dass handelsübliche, blattartig ausgestaltete chirurgische Werkzeuge in der Einspannvorrichtung einspannbar sind.

In wiederum einer anderen Ausführungsform sind die erste und die zweite Einspannvorrichtung mittels axialem Verschieben einer Hülse verriegel- und entriegelbar. Hierdurch ist der Vorteil erreichbar, dass die Kupplungsvorrichtung einfach ausgestaltet werden kann. Vorzugsweise wird die Hülse mittels einer Druckfeder in der axialen Position mit verriegelten Einspannvorrichtungen gehalten. Damit ist eine einfache Bedienung, d.h. Verriegelung oder Entriegelung der Einspannvorrichtungen erreichbar.

In einer weiteren Ausführungsform ist die erste Einspannvorrichtung derart ausgebildet, dass eine bezüglich der Längsachse rotativ und axial formschlüssige Verbindung mit einem Werkzeug herstellbar ist. Vorzugsweise ist auch die zweite Einspannvorrichtung derart ausgebildet, dass ein bezüglich der Längsachse rotativer und axialer Formschluss mit einem Werkzeug erreichbar ist. Damit ist eine rigide Fixierung des Werkzeugs in jeder der zwei Einspannvorrichtung gewährleistet.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 einen Längsschnitt durch eine Ausführungsform der erfindungsgemässen Kupplungsvorrichtung mit entriegelten Einspannvorrichtungen;
Fig. 2 einen Längsschnitt durch die in Fig. 1 dargestellte Ausführungsform der erfindungsgemässen Kupplungsvorrichtung mit verriegelten Einspannvorrichtungen und einem in der ersten Einspannvorrichtung eingespannten chirurgischen Sägeblatt;
Fig. 3 einen Längsschnitt durch die in den Fig. 1 und 2 dargestellte Ausführungsform der erfindungsgemässen Kupplungsvorrichtung mit verriegelten Einspannvorrichtungen und einem in der zweiten Einspannvorrichtung eingespannten chirurgischen Sägeblatt;
Fig. 4 eine Seitenansicht eines mandibula Sägeblattes mit einem zapfenförmigen Einspannsegment;
Fig. 5 eine Seitenansicht eines crescentic Sägeblattes mit einem zapfenförmigen Einspannsegment; und
Fig. 6 eine Draufsicht auf ein sagittales Sägeblatt mit einem ebenen U-förmigen Einspannsegment.

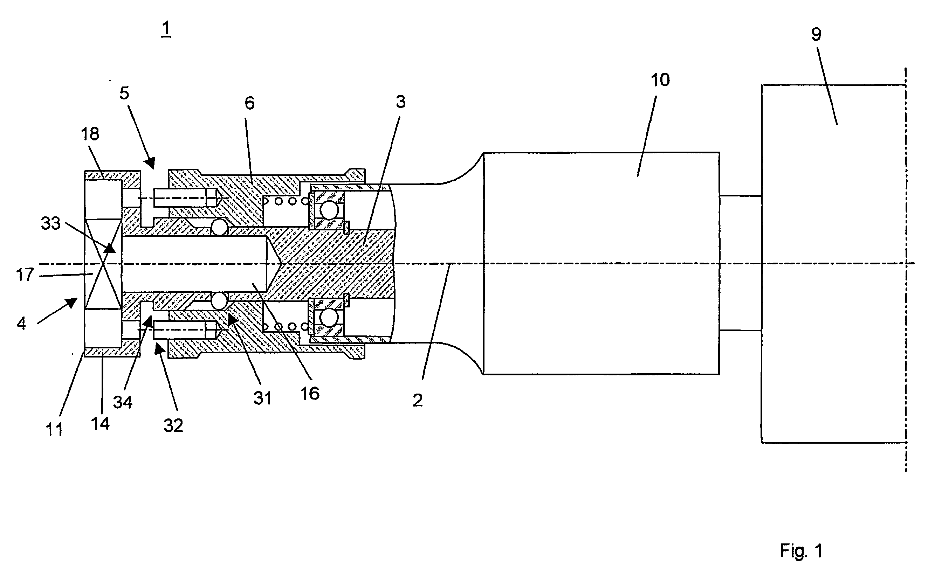

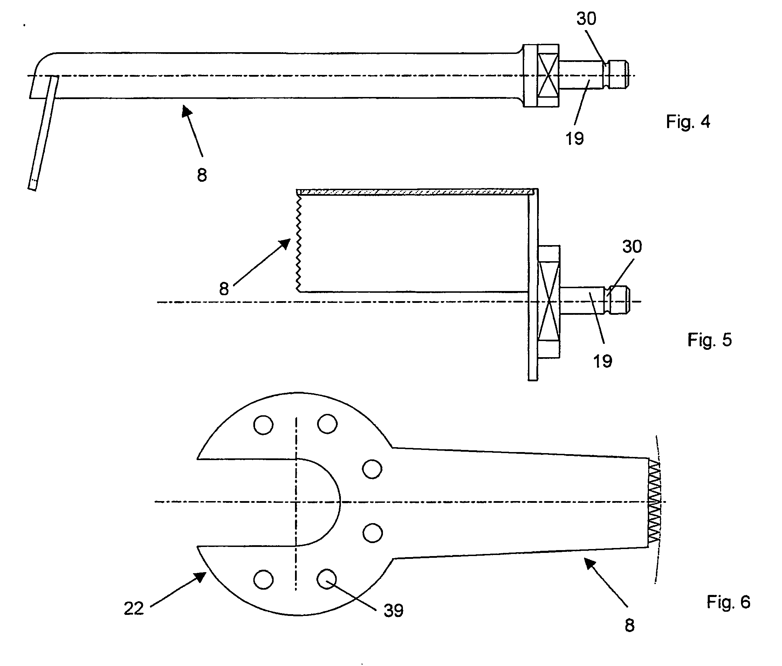

In den Fig. 1 bis 3 ist eine Ausführungsform dargestellt, welche eine zu einer Längsachse 2 koaxiale Kupplungsvorrichtung 1 mit einer ersten Einspannvorrichtung 4 für Werkzeuge 8 gemäss den Fig. 4 und 5 und einer zweiten Einspannvorrichtung 5 für Werkzeuge 8 gemäss Fig. 6 umfasst. Die zwei Einspannvorrichtungen 4;5 sind verschieden ausgebildet. Die erste Einspannvorrichtung 4 umfasst eine erste, koaxiale Aufnahme 33 für ein zapfenförmiges Einspannsegment 19 (Fig. 4 und 5) eines Werkzeuges 8 während die zweite Einspannvorrichtung 5 eine zweite, auf der Peripherie der Kupplungsvorrichtung 1 angeordnete Aufnahme 34 für ein ebenes U-förmiges Einspannsegment 22 (Fig. 6) umfasst.

Als Werkzeug 8 (Fig. 4 bis 6) sind hier verschiedene Ausführungsformen von Sägeblättern dargestellt, wobei in Fig. 4 ein mandibula Sägeblatt, in Fig. 5 ein crescentic Sägeblatt und in Fig. 6 ein sagittales Sägeblatt abgebildet sind. Diese werden je nach Ausgestaltung des Einspannsegmentes 19;22 in die erste oder zweite Aufnahme 33;34 der zugehörigen Einspannvorrichtung 4;5 eingeführt und mittels ersten respektive zweiten Verriegelungsmitteln 31;32 in der Kupplungsvorrichtung 1 fixiert. Durch die Kupplungsvorrichtung 1 wird das eingespannte Werkzeug 8 mit einer Antriebswelle 3 verbunden. In der hier dargestellten Ausführungsform wird die Antriebswelle 3 mittels einem axial zwischen Antriebsmotor 9 und Kupplungsvorrichtung 1 angeordnetem Getriebe 10 um die Längsachse 2 rotativ oszillierend angetrieben.

Beide Einspannvorrichtungen 4;5 werden simultan durch axiales Verschieben derselben Hülse 6 verriegelt oder entriegelt. In Fig. 1 ist die Hülse 6, welche zur Bedienung der zwei Einspannvorrichtungen 4;5 dient, in ihrer hinteren Position dargestellt In dieser hinteren Position der Hülse 6 sind die zwei Einspannvorrichtungen 4;5 entriegelt, so dass ein Werkzeug 8 mit einem zapfenförmigen Einspannsegment 19 in die erste Einspannvorrichtung 4 einführbar ist oder ein Werkzeug 8 mit einem ebenen U-förmigen Einspannsegment 22 in die zweite Einspannvorrichtung 5 vom einführbar ist.

Die erste Einspannvorrichtung 4 umfasst am freien Ende 11 der Kupplungsvorrichtung 1 eine erste rotative Mitnahme 14 mit einem am freien Ende 11 der Kupplungsvorrichtung 1 offenen, koaxialen Hohlraum 13 zur Aufnahme des zapfenförmigen Einspannsegmentes 19 eines Werkzeuges 8. Der Hohlraum 13 hat am freien Ende 11 der Kupplungsvorrichtung 1 ein Hohlraumsegment 38 mit einer zur Längsachse 2 orthogonalen unrunden Querschnittsfläche und axial angrenzend eine kreiszylindrische Zentralbohrung 16. Das aussenliegende Hohlraumsegment 38 besteht aus einer ovalen Öffnung mit zwei parallelen Seitenflächen 17 und zwei zur Längsachse 2 konzentrisch bogenförmigen Seitenflächen 18, so dass eine bezüglich Rotation um die Längsachse 2 formschlüssige Verbindung zwischen der ersten rotativen Mitnahme 14 und einem komplementär ausgebildeten, zapfenförmigen Einspannsegment 19 eines Werkzeuges 8 (Fig. 4 und 5) herstellbar ist. Die ersten Verriegelungsmittel 31 der ersten Einspannvorrichtung 4 umfassen Kugeln 21, welche in Bohrungen 23 mit orthogonal zur Längsachse 2 stehenden Bohrungsachsen 24 quer zur Längsachse 2 verschiebbar gelagert sind. Die Kugeln 21 sind durch Anschrägungen 26 an den inneren Enden 27 der zur Längsachse 2 parallelen Führungsnuten 28 in der Bohrung 7 der Hülse 6 bewegbar und je nach axialer Position der Hülse 6 in eine ringförmige Nute 30 am zapfenförmigen Einspannsegment 19 eines Werkzeuges 8 (Fig. 4 und 5) einrastbar oder ausrastbar. Die Hülse 6 wird mittels einer koaxial in der Bohrung 7 der Hülse 6 angeordneten und axial an der Antriebswelle 3 abgestützten Druckfeder 15 gegen das freie Ende 11 der Kupplungsvorrichtung 1 gedrückt und in ihrer vorderen Position gehalten. In dieser vorderen Position sind beide Einspannvorrichtungen 4;5 verriegelt, d.h. bei der ersten Einspannvorrichtung 4 werden die Kugeln 21 in ringförmige Nute 30 am zapfenförmigen Einspannsegment 19 eines Werkzeuges 8 gepresst, wodurch das Werkzeug 8 axial formschlüssig fixiert wird. Damit die Kugeln 21 bei nicht eingeführtem zapfenförmigen Verbindungssegment 19 des Werkzeuges 8 nicht aus den Bohrungen 23 herausfallen können, sind die Bohrungen 23 beim Eintritt in die Zentralbohrung 16 verengt.

Die zweite Einspannvorrichtung 5 umfasst einen vom freien Ende 11 der Kupplungsvorrichtung 1 axial zurückversetzt angeordnete, zweite rotative Mitnahme 20 mit den zweiten Verriegelungsmitteln 32. Diese zweiten Verriegelungsmittel 32 bestehen im wesentlichen aus Stiften 29, deren Achsen auf einer zur Längsachse 2 konzentrischen Kreiszylindermantelfläche angeordnet sind. Die Stifte 29 sind mit ihren einen festen Enden 35 in Bohrungen 25 am vorderen Ende 37 in der Hülse 6 eingepresst, während die freien Enden 36 der Stifte 29 bei verriegelten zweiten Verriegelungsmitteln 32 in fluchtende Bohrungen 38 an der ersten rotativen Mitnahme 14 eingreifen. Die Stifte 29 werden beim Verriegeln der Einspannvorrichtung 5 durch komplementäre Bohrungen 39 im ebenen U-förmigen Einspannsegment 22 (Fig. 6) des Werkzeuges 8 geschoben, so dass dieses rotativ formschlüssig mit der Antriebswelle 3 verbunden ist. In axialer Richtung wird das ebene U-förmige Einspannsegment 22 des Werkzeuges 8 zwischen der ersten rotativen Mitnahme 14 und dem vorderen Ende 37 der Hülse 6 fixiert.

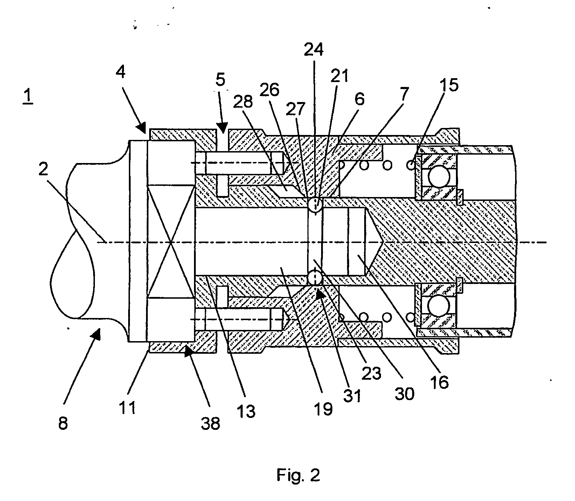

In Fig. 2 wird die Kupplungsvorrichtung 1 im verriegeltem Zustand mit einem eingespannten Werkzeug 8 dargestellt, wobei das Werkzeug 8 ein zapfenförmiges Einspannsegment 19 aufweist, welches in der ersten Einspannvorrichtung 4 fixiert ist. Die Übertragung des Drehmomentes um die Längsachse 2 von der Antriebswelle 3 auf das Werkzeug 8 wird durch die rotative Mitnahme 14 übernommen. Mittels der zwei im Hohlraumsegment 38 der rotativen Mitnahme 14 angeordneten, parallelen Seitenfläche 17 wird eine rotativ formschlüssige Verbindung zwischen der rotativen Mitnahme 14 und dem ersten Einspannsegment 19 des Werkzeuges 8 hergestellt. Die axiale Fixierung des ersten Einspannsegments 19 innerhalb der ersten Einspannvorrichtung 4 ist durch die in die peripher am zapfenförmigen Einspannsegment 19 angebrachte Nute 30 eingreifenden Kugeln 21 axial formschlüssig.

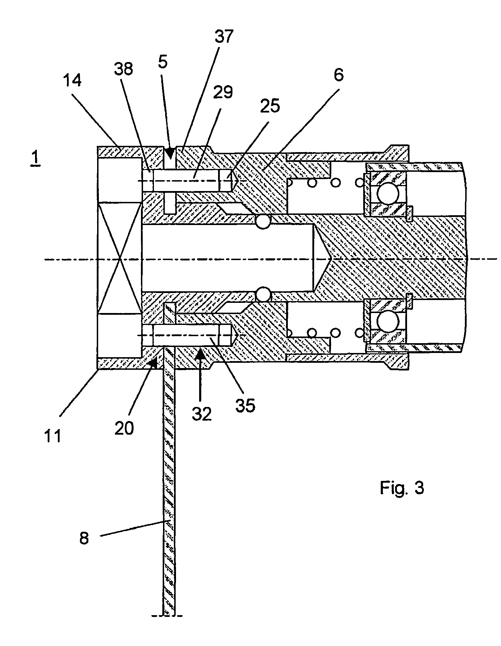

In Fig. 3 wird die Kupplungsvorrichtung 1 ebenfalls im verriegelten Zustand mit einem eingespannten Werkzeug 8 dargestellt, wobei das Werkzeug 8 ein ebenes U-förmiges Einspannsegment 22 aufweist, welches in der zweiten Einspannvorrichtung 5 fixiert ist.

## Patentansprüche

1. Kupplungsvorrichtung (1) zur Verbindung eines Werkzeuges mit einer eine Längsachse (2) aufweisenden Antriebswelle (3), umfassend
A) eine zur Längsachse (2) koaxial angeordnete, mit der Antriebswelle (3) verbundene erste Einspannvorrichtung (4) mit ersten Verriegelungsmitteln (31) zur lösbar fixierbaren Aufnahme eines Werkzeuges (8);
B) eine koaxial angeordnete, die Einspannvorrichtung (4) auf einem Teil ihrer axialen Länge umschliessende und axial bewegbare Hülse (6) zur Verriegelung oder Entriegelung der ersten Verriegelungsmittel (31),
**dadurch gekennzeichnet, dass**
C) die Kupplungsvorrichtung (1) zusätzlich eine zur Längsachse (2) koaxial angeordnete, mit der Antriebswelle (3) verbundene, zweite Einspannvorrichtung (5) mit zweiten Verriegelungsmitteln (32) zur lösbar fixierbaren Aufnahme eines Werkzeuges (8) umfasst.

2. Kupplungsvorrichtung (1) nach Anspruch 1, **dadurch gekennzeichnet, dass** die erste Einspannvorrichtung (4) ein zur Längsachse (2) koaxiales Einführen eines Werkzeuges (8) gestattet.

3. Kupplungsvorrichtung (1) nach Anspruch 1 oder 2, **dadurch gekennzeichnet, dass** die zweite Einspannvorrichtung (5) ein quer zur Längsachse (2) gerichtetes Einführen eines Werkzeuges (8) gestattet.

4. Kupplungsvorrichtung (1) nach einem der Ansprüche 1 bis 3, **dadurch gekennzeichnet, dass** die erste Einspannvorrichtung (4) und die zweite Einspannvorrichtung (5) mittels axialem Verschieben der Hülse (6) verriegel- und entriegelbar sind.

5. Kupplungsvorrichtung (1) nach Anspruch 4, **dadurch gekennzeichnet, dass** die Hülse (6) mittels einer Druckfeder (15) in der axialen Position mit verriegelten Einspannvorrichtungen (4;5) Position gehalten wird.

6. Kupplungsvorrichtung (1) nach einem der Ansprüche 1 bis 5, **dadurch gekennzeichnet, dass** die erste Einspannvorrichtung (4) eine bezüglich der Längsachse (2) rotativ und axial formschlüssige Verbindung mit einem Werkzeug (8) gestattet.

7. Kupplungsvorrichtung (1) nach einem der Ansprüche 1 bis 6, **dadurch gekennzeichnet, dass** die zweite Einspannvorrichtung (5) mindestens eine bezüglich der Längsachse (2) rotativ formschlüssige Verbindung mit einem Werkzeug (8) gestattet.

8. Kupplungsvorrichtung (1) nach einem der Ansprüche 1 bis 7, **dadurch gekennzeichnet, dass** sie zusätzlich ein Werkzeug (8), vorzugsweise ein Sägewerkzeug umfasst.

## Claims

1. Coupling device (1) for connecting a tool with a drive shaft (3) having a longitudinal axis (2), comprising
A) a first clamping device (4), disposed coaxially with the longitudinal axis (2) and connected with the drive shaft (3), and having first locking means (31) for the detachably fixable seating of a tool (8);
B) a coaxially disposed sleeve (6), which surrounds the clamping device (4) on a portion of its axial length and is axially movable, for locking or unlocking the first locking means (31),
**characterized in that**
C) the coupling device (1) additionally comprises a second clamping device (5), which is disposed coaxially with the longitudinal axis (2) and connected with the drive shaft (3), and with second locking means (32) for the detachably fixable seating of a tool (8).

2. The coupling device (1) of claim 1, **characterized in that** the first clamping device (4) permits a tool to be introduced coaxially with the longitudinal axis (2).

3. The coupling device (1) of claims 1 or 2, **characterized in that** the second clamping device (5) permits a tool (8) to be introduced transversely to the longitudinal axis (2).

4. The coupling device (1) of one of the claims 1 to 3, **characterized in that** the first clamping device (4) and the second clamping device (5) can be locked and unlocked by shifting the sleeve (6) axially.

5. The coupling device (1) of claim 4, **characterized in that** the sleeve (6) is held by a compression spring (15) in the axial position with locked clamping devices (4; 5).

6. The coupling device (1) of one of the claims 1 to 5, **characterized in that** the first locking device (4) permits a connection with a tool (8), which is rotative with respect to the longitudinal axis (2) and positive axially.

7. The coupling device (1) of one of the claims 1 to 6, **characterized in that** the second clamping device (5) permits at least one connection with a tool (8), the connection being rotatively positive with respect to the longitudinal axis (2).

8. The coupling device (1) of one of the claims 1 to 7, **characterized in that** it additionally comprises a tool (8), preferably a sawing tool.

## Revendications

1. Dispositif d'accouplement (1) pour relier un outil à un arbre d'entraînement (3) ayant un axe longitudinal (2), comprenant
A) un premier dispositif de serrage (4) relié à l'arbre d'entraînement (3), disposé coaxialement à l'axe longitudinal (2), comprenant des premiers moyens de verrouillage (31) pour loger par fixation amovible un outil (8) ;
B) une douille (6) disposée coaxialement, qui entoure le dispositif de serrage (4) sur une partie de sa longueur axiale et qui est mobile dans le sens axial, pour verrouiller ou déverrouiller le premier moyen de verrouillage (31),
**caractérisé en ce que**
C) le dispositif d'accouplement (1) comprend en outre un deuxième dispositif de serrage (5) relié à l'arbre d'entraînement (3), disposé coaxialement à l'axe longitudinal (2), comprenant des deuxièmes moyens de verrouillage (32) pour loger par fixation amovible un outil (8).

2. Dispositif d'accouplement (1) selon la revendication 1, **caractérisé en ce que** le premier dispositif de serrage (4) permet d'introduire un outil (8) coaxialement à l'axe longitudinal (2).

3. Dispositif d'accouplement (1) selon la revendication 1 ou 2, **caractérisé en ce que** le deuxième dispositif de serrage (5) permet d'introduire un outil (8) transversalement à l'axe longitudinal (2).

4. Dispositif d'accouplement (1) selon l'une quelconque des revendications 1 à 3, **caractérisé en ce que** le premier dispositif de serrage (4) et le deuxième dispositif de serrage (5) peuvent être verrouillés et déverrouillés par un coulissement axial de la douille (6).

5. Dispositif d'accouplement (1) selon la revendication 4, **caractérisé en ce que** la douille (6) est maintenue, au moyen d'un ressort de compression (15), en position axiale avec les dispositifs de serrage (4 ; 5) en position verrouillée.

6. Dispositif d'accouplement (1) selon l'une quelconque des revendications 1 à 5, **caractérisé en ce que** le premier dispositif de serrage (4) permet une liaison par emboîtement rotatif et axial par rapport à l'axe longitudinal (2) avec un outil (8).

7. Dispositif d'accouplement (1) selon l'une quelconque des revendications 1 à 6, **caractérisé en ce que** le deuxième dispositif de serrage (5) permet au moins une liaison par emboîtement rotatif par rapport à l'axe longitudinal (2) avec un outil (8).

8. Dispositif d'accouplement (1) selon l'une quelconque des revendications 1 à 7, **caractérisé en ce qu'**il comprend en outre un outil (8), de préférence un outil de sciage.
